# EUROPÄISCHE PATENTSCHRIFT

(11) **EP 0 705 278 B1**
(45) Veröffentlichungstag und Bekanntmachung des Hinweises auf die Patenterteilung: **26.11.2003**
(21) Anmeldenummer: 95909730.4
(22) Anmeldetag: 16.02.1995
(51) Int. Cl.: C07K 14/715, C07K 16/28, C12N 15/62, A61K 39/395, A61K 39/42, A61K 38/17, A61K 38/55, A61K 31/37, A61K 31/70

(54) **HEMMER VON APOPTOSE**
APOPTOSE INHIBITOR
INHIBITEUR D'APOPTOSE

(30) Priorität: 08.04.1994 DE 4412177
(43) Veröffentlichungstag der Anmeldung: 10.04.1996
(73) Patentinhaber: Deutsches Krebsforschungszentrum Stiftung des öffentlichen Rechts, 69120 Heidelberg (DE)
(72) Erfinder: KRAMMER, Peter, D-69120 Heidelberg (DE); WESTENDORP, Michael, D-69120 Heidelberg (DE); SCHULZE-OSTHOFF, Klaus, D-69221 Dossenheim (DE); DEBATIN, Klaus-Michael, D-69120 Heidelberg (DE); FRANK, Rainer, D-69120 Heidelberg (DE); DHEIN, Jens, D-68535 Edingen-Neckarhausen (DE); WALCZAK, Henning, D-69124 Heidelberg (DE); KNIPPING, Eckart, D-69120 Heidelberg (DE); STRICKER, Kirstin, D-69120 Heidelberg (DE)
(74) Vertreter: Schüssler, Andrea, Dr.
(86) Internationale Anmeldenummer: EP9500573
(87) Internationale Veröffentlichungsnummer: WO95027735

(56) Entgegenhaltungen:
- EP-A- 0 344 006
- WO-A-91/15224
- CELL., Bd.75, 19. November 1993, CAMBRIDGE, NA US Seiten 653 - 660 M. MIURA ET AL. 'INDUCTION OF APOPTOSIS IN FIBROBLASTS BY IL-1BETA-CONVERTING ENZYME, A MAMMALIAN HOMOLOG OF THE C. ELEGANS CELL DEATH GENE ced-3.'
- JOURNAL OF IMMUNOLOGY., Bd.149, Nr.10, 15. November 1992, BALTIMORE US Seiten 3166 - 3173 J. DHEIN ET AL. 'INDUCTION OF APOPTOSIS BY MONOCLONAL ANTIBODY ANTI-APO-1 CLASS SWITH VARIANTS IS DEPEDENT ON CROSS-LINKING OF APO-1 CELL SURFACE ANTIGENS.' in der Anmeldung erwähnt
- THE JOURNAL OF EXPERIMENTAL MEDICINE, Bd.179, Nr.3, 1. März 1994, NEW YORK, N.Y., US Seiten 873 - 879 T. SUDA ET AL. 'PURIFICATION AND CHARACTERIZATION OF THE FAS-LIGAND THAT INDUCES APOPTOSIS.' in der Anmeldung erwähnt
- JOURNAL OF IMMUNOLOGY., Bd.146, Nr.12, 15. Juni 1991, BALTIMORE US Seiten 4325 - 4332 M.R.POSNER ET AL. 'AN IgG HUMAN MONOCLONAL ANTIBODY THAT REACTS WITH HIV-1/GP120, INHIBITS VIRUS BINDING TO CELLS, AND NEUTRALIZES INFECTION.'
- CELL., Bd.78, 29. Juli 1994, CAMBRIDGE, NA US Seiten 343 - 352 N.P.C. WALKER ET AL. 'CRYSTAL STRUCTURE OF THE CYSTEINE PROTEASE INTERLEUKIN-1BETA-CONVERTING ENZYME: A (P20/P10)2 HOMODIMER.'

## Beschreibung

Die Erfindung betrifft Mittel zur Hemmung von Apoptose. Ferner betrifft die Erfindung Verbindungen, die sich zur Hemmung von Apoptose eignen, sowie Verfahren zu deren Herstellung.

Apoptose ist die Bezeichnung für programmierten Zelltod. Dieser findet sich, z.B. bei der Organentwicklung und Metamorphose, der Gewebeatrophie und Tumorregression. Apoptose ist mit einer Kondensation des Cytoplasmas, einem Verlust von Plasmamembranvilli, einer Segmentation des Kerns und extensivem Abbau chromosomaler DNA verbunden (vgl. Oehm, A. et al., The Journal of Biological Chemistry, Band 267, Nr. 15 (1992), Seiten 10709-10715).

In Zellen, die für Apoptose positiv sind, findet sich oftmals ein mit APO-1 bezeichnetes Zell-Oberflächenprotein. Dieses Glycoprotein wird der Tumornekrosefaktor/Nervenwachstumsfaktor-Rezeptor-Familie zugerechnet. Durch Crosslinking von APO-1 über einen anti-APO-1-Antikörper wird Apoptose bei den genannten Zellen induziert (vgl. Oehm, A. et al., supra). Gleiches scheint auch durch Bindung eines mit APO-1-Ligand bezeichneten löslichen oder Membran-gebundenen Proteins an APO-1 bewirkt zu werden (vgl. Suda, T. und Nagata, S., J. Exp. Med., The Rockefeller University Press, Band 179 (1994), Seiten 873-879). In Dhein, J. et al., The Journal of Immunology, Band 149 , Nr. 10 (1992), S. 3166-3173 ist ein anti-APO-1-F(ab)₂ - Fragment beschrieben.

Über die Hemmung von Apoptose ist dagegen nichts bekannt. Dies wäre aber umso notwendiger, da jüngste Arbeiten des Anmelders zeigen, daß Apoptose auch bei verschiedenen Erkrankungen, wie AIDS und Autoimmunerkrankungen, auftritt. Bei AIDS scheint Apoptose für die starke Abnahme der CD4-T Zellen verantwortlich zu sein.

Der vorliegenden Erfindung liegt somit die Aufgabe zugrunde, ein Mittel bereitzustellen, mit dem Apoptose gehemmt werden kann.

Erfindungsgemäß wird dies erreicht durch Verwendung einer oder mehrerer den APO-1-Ligand hemmenden bzw. abfangenden Verbindungen, wobei die Verbindungen in einem Individuum nicht als fremd angesehen werden und ausgewählt sind aus:
- einem anti-APO-1-Ligand-Antikörper,
- einem APO-1,
- einer extrazellulären APO-1-Domäne,
- einer Verbindung mit mindestens einer extrazellulären APO-1-Domäne und einem Träger,
- einem eine APO-1-Ligand-Bindungsstelle aufweisenden Peptid, und
- einer Verbindung mit mindestens einem eine APO-1-Ligand-Bindungsstelle aufweisenden Peptid und einem Träger
zur Herstellung eines Arzneimittels zur Hemmung von Apoptose bei einer mit einer HIV-Infektion assoziierten Erkrankung oder Autoimmunerkrankung.

Die Herstellung einer solchen Verbindung erfolgt in üblicher Weise. Der Fachmann wird zur Herstellung eines anti-APO-1-Ligand-Antikörpers z.B. von dem in Suda, T. und Nagata, S., supra beschriebenen APO-1-Ligand ausgehen. Er wird darauf achten, daß der Fc-Teil des Antikörpers in einem Individuum nicht als fremd angesehen wird. Verfahren, die dies ermöglichen, sind ihm bekannt. Ferner wird der Fachmann hinsichtlich der Herstellung von APO-1 bzw. einer extrazellulären APO-1-Domäne z.B. von dem in der EP-92 107 060.3 beschriebenen APO-1 bzw. seiner extrazellulären Domäne ausgehen. Desweiteren wird er zur Herstellung eines eine APO-1-Ligand-Bindungsstelle aufweisenden Peptids z.B. die Kombination aus vorstehendem APO-1-Ligand und vorstehendem APO-1 bzw. seiner extrazellulären Domäne nutzen.

Die Herstellung einer Verbindung mit mindestens einer extrazellulären APO-1-Domäne und einem Träger wird nachstehend beispielhaft beschrieben. In analoger Weise kann eine Verbindung mit mindestens einem eine APO-1-Ligand-Bindungsstelle aufweisenden Peptid und einem Träger hergestellt werden.

Ein weitere Aspekt der Erfinding betrifft ein Fusionsprotein, bei dem eine extrazelluläre APO-1 Domäne über eine Antikörper-Hinge-Region an einen Fc-Teil eines humanen Antikörpers fusioniert ist, wobei die APO-1 Domäne aus den Aminosäuren 1 - 171 des humanen APO-1 Moleküls besteht.

Erfindungsgemäß wird vorstehendes Mittel besonders zur Hemmung von Apoptose bei einer mit einer HIV-Infektion assoziierten Erkrankung eingesetzt. Als besonders vorteilhaft erweist sich das Mittel dabei, wenn es ferner eine bis alle Komponenten enthält von:
(a) eine den TAT-Rezeptor hemmende Verbindung,
(b) eine TAT hemmende bzw. abfangende Verbindung,
(c) eine den intrazellulären TAT-Rezeptor-Signalweg hemmende Verbindung,
(d) eine den CD4-Rezeptor hemmende Verbindung,
(e) eine gp120 hemmende bzw. abfangende Verbindung, und
(f) eine den intrazellulären CD4-Rezeptor-Signalweg hemmende Verbindung, wobei die Komponente(n) in einem Individuum nicht als fremd angesehen wird(werden).

Der Ausdruck "eine den TAT-Rezeptor hemmende Verbindung" umfaßt jegliche zur Hemmung des TAT-Rezeptors geeignete Verbindung. Vorzugsweise ist dies ein anti-TAT-Rezeptor-Antikörper. Die Herstellung eines solchen erfolgt in üblicher Weise. Der Fachmann wird darauf achten, daß der Fc-Teil des Antikörpers in einem Individuum nicht als fremd angesehen wird. Verfahren, die dies ermöglichen, sind dem Fachmann bekannt.

Als weitere bevorzugte "den TAT-Rezeptor hemmende Verbindung" ist ein TAT-Analogon zu nennen. Ein solches bindet nach wie vor an den TAT-Rezeptor, induziert jedoch nicht mehr den intrazellulären TAT-Rezeptor-Signa!weg. Die Herstellung eines solchen Analogons erfolgt in üblicher Weise, wobei der Fachmann z.B. von dem in Arya, S., K., et al., Science, Band 229 (1985), Seiten 69-73 beschriebenen TAT ausgeht.

Der Ausdruck "eine TAT-hemmende bzw. abfangende Verbindung" umfaßt jegliche zur Hemmung bzw. zum Abfangen von TAT geeignete Verbindung. Vorzugsweise ist dies eine der folgenden Verbindungen:
- ein anti-TAT-Antikörper,
- ein TAT-Rezeptor,
- eine extrazelluläre TAT-Rezeptor-Domäne,
- eine Verbindung mit mindestens einer extrazellulären TAT-Rezeptor-Domäne und einem Träger,
- ein eine TAT-Bindungsstelle aufweisendes Peptid,
- eine Verbindung mit mindestens einem eine TAT-Bindungsstelle aufweisenden Peptid und einem Träger,
- eine TAT-Bindungsstelle auf Nukleinsäurebasis, und
- eine transdominante TAT-Mutante.

Die Herstellung einer solchen Verbindung erfolgt in üblicher Weise. Der Fachmann wird zur Herstellung eines anti-TAT-Antikörpers z.B. von dem in Arya, S., K. et al., supra beschriebenen TAT ausgehen. Er wird darauf achten, daß der Fc-Teil des Antikörpers in einem Individuum nicht als fremd angesehen wird. Verfahren, die das ermöglichen, sind ihm bekannt. Ferner wird er hinsichtlich einer TAT-Bindungsstelle auf Nukleinsäurebasis z.B. von der in Feng, S. und Holland, E., C., Nature, Band 334 (1988), Seiten 165-167 beschriebenen TAT-LTR-Sequenz ausgehen. Desweiteren wird der Fachmann bezüglich einer transdominanten TAT-Mutante z.B. von der in Echetebu, C., O. und Rice, A., P., J. Acquir. Immune Def. Syndrome, Band 6, (1993), Seiten 550-557 beschriebenen Mutante ausgehen.

Der Ausdruck "eine den intrazellulären TAT-Rezeptor-Signalweg hemmende Verbindung" umfaßt jegliche zur Hemmung des intrazellulären TAT-Rezeptor-Signalwegs geeignete Verbindung.

Der Ausdruck "eine den CD4-Rezeptor hemmende Verbindung" umfaßt jegliche zur Hemmung des CD4-Rezeptors geeignete Verbindung. Vorzugsweise ist dies ein anti-CD4-Rezeptor-Antikörper. Die Herstellung eines solchen erfolgt in üblicher Weise, wobei der Fachmann z.B. von dem in Capon, D., J. und Ward, R., H., R., Annu. Rev. Immunol. 9, (1991), Seiten 649-678, beschriebenen CD4-Rezeptor ausgeht. Der Fachmann wird darauf achten, daß der Fc-Teil des Antikörpers in einem Individuum nicht als fremd angesehen wird. Verfahren, die das ermöglichen, sind dem Fachmann bekannt.

Als weitere bevorzugte "den CD4-Rezeptor hemmende Verbindung" ist ein gp 120-Analogon zu nennen. Ein solches bindet nach wie vor an den CD4-Rezeptor, induziert jedoch nicht mehr den intrazellulären CD4-Rezeptor-Signalweg. Die Herstellung eines solchen Analogons erfolgt in üblicher Weise, wobei der Fachmann z.B. von dem in Capon, D., J. und Ward, R., H., R., supra beschriebenen gp 120 ausgeht.

Der Ausdruck "eine gp 120 hemmende bzw. abfangende Verbindung" umfaßt jegliche zur Hemmung bzw. Abfangung von gp 120 geeignete Verbindung. Vorzugsweise ist dies eine der folgenden Verbindungen:
- ein anti-gp 120-Antikörper,
- ein CD4-Rezeptor,
- eine extrazelluläre CD4-Rezeptor-Domäne,
- eine Verbindung mit mindestens einer extrazellulären CD4-Rezeptor-Domäne und einem Träger,
- ein eine gp 120-Bindungsstelle aufweisendes Peptid, und
- eine Verbindung mit mindestens einem eine gp 120-Bindungsstelle aufweisenden Peptid und einem Träger.

Die Herstellung einer solchen Verbindung erfolgt in üblicher Weise. Der Fachmann wird zur Herstellung eines anti-gp 120-Antikörpers z.B. von dem in Capon, D.J. und Ward, R., H., R., supra beschriebenen gp 120 ausgehen. Er wird darauf achten, daß der Fc-Teil des Antikörpers in einem Individuum nicht als fremd angesehen wird. Verfahren, die das ermöglichen, sind ihm bekannt. Ferner wird der Fachmann hinsichtlich der Herstellung eines CD4-Rezeptors bzw. einer extrazellulären CD4-Rezeptor-Domäne z.B. von dem in Capon, D., J. und Ward, R., H., R., supra beschriebenen CD4-Rezeptor bzw. seiner extrazellulären Domäne ausgehen. Desweiteren wird er zur Herstellung eines eine gp 120-Bindungsstelle aufweisenden Peptids z. B. die Kombination aus vorstehendem gp 120 und vorstehendem CD4-Rezeptor bzw. seiner extrazellulären Domäne nutzen.

Die Herstellung einer Verbindung mit mindestens einer extrazellulären CD4-Rezeptor-Domäne bzw. einem mindestens eine gp 120-Bindungsstelle aufweisenden Peptid und einem Träger kann in analoger Weise erfolgen, wie nachstehend für eine Verbindung mit mindestens einer extrazellulären APO-1-Domäne und einem Träger beschrieben.

Der Ausdruck "eine den intrazellulären CD4-Rezeptor-Signalweg hemmende Verbindung" umfaßt jegliche zur Hemmung des intrazellulären CD4-Rezeptor-Signalwegs geeignete Verbindung.

Es wird verstanden, daß ein vorstehendes Mittel ein bis mehrere Verbindungen einer einzelnen Komponente aufweisen kann.

Erfindungsgemäß wird auch eine Verbindung mit mindestens einer extrazellulären APO-1-Domäne und einem Träger bereitgestellt, wobei die Domäne(n) und der Träger in einem Individuum nicht als fremd angesehen werden.

Der Ausdruck "Träger" umfaßt jegliche Verbindung, an die ein oder mehrere extrazelluläre APO-1 Domänen gebunden sein können.

In bevorzugter Ausführungsform ist der Träger ein Protein, z. B. Serumalbumin, Hämoglobin, Fibrinogen, Kollagen oder ein Fc-Teil eines Antikörpers, wobei letzteres bevorzugt ist. In ganz bevorzugter Ausführungsform ist die erfindungsgemäße Verbindung ein Fusionsprotein.

Eine erfindungsgemäße Verbindung kann in üblicher Weise hergestellt werden. Im Falle eines Fusionsproteins erweist sich das folgende Herstellungsverfahren als günstig:
(a) Amplifikation einer DNA durch übliche PCR-Technik, wobei die DNA für mindestens eine extrazelluläre APO-1-Domäne und eine am 3'-Ende dieser angebrachte Bindungsregion codiert,
(b) Amplifikation einer DNA durch übliche PCR-Technik, wobei die DNA für einen Protein-Träger und eine am 5'-Ende des Protein-Trägers angebrachte Bindungsregion codiert,
(c) Vereinigung der amplifizierten DNAs von (a) und (b) und weitere gemeinsame Amplifikation dieser durch übliche PCR-Technik, wobei ein dem 5'-Ende der DNA der APO-1-Domäne entsprechender Primer und ein dem 3'-Ende der DNA des Protein-Trägers entsprechender Primer verwendet werden, wodurch ein amplifiziertes, beide DNAs in Fusion enthaltendes DNA-Fragment erhalten wird, und
(d) Insertion des DNA-Fragments von (c) in einen Expressionsvektor und Expression des DNA-Fragments in üblicher Weise.

In bevorzugter Ausführungsform ist die Bindurigsregion von (a) und (b) eine Antikörper-Hinge-Region oder ein Teil davon. Ferner kann sie auch eine Thrombinspaltstelle sein.

In vorstehendem Herstellungsverfahren werden verschiedene DNAs durch übliche PCR-Technik amplifiziert. Als DNA, die für mindestens eine extrazelluläre APO-1-Domäne codiert, wird der Fachmann z.B. die in der EP-92 107 060.3, supra beschriebene DNA als Basis verwenden. Dieser wird er am 3'Ende eine für eine Bindungsregion codierende DNA anfügen. Im Falle einer für eine Hinge-Region eines humanen Antikörpers oder einen Teil davon codierenden DNA wird der Fachmann z.B. auf die in Dübel, S., et al., Methods in Molecular and Cellular Biology, Band 3 (1992), Seiten 47-52 beschriebene DNA zurückgreifen. Hinsichtlich der für den Protein-Träger, z.B. Fc-Teil eines humanen Antikörpers, codierenden DNA wird der Fachmann z.B. ebenso auf die in Dübel, S. et al., supra beschriebene DNA zurückgreifen.

Die Expression des amplifizierten DNA-Fragments erfolgt in üblichen Vektoren, wie pCDN83, pCEV4 und pCDM8 zur Expression in tierischen Zellen, pGEMEX und pUC zur Expression in E. coli., sowie pY100 und YCpAD1 zur Expression in Hefe. Als tierische Zellen eignen sich insbesondere L-, COS- und CHO-Zellen, während als prokaryotische Mikroorganismen insbesondere E. coli-Stämme und als Hefezellen besonders solche von Saccharomyces und Pichia pastoris zu nennen sind.

Die vorliegende Erfindung eröffnet einen neuen Weg, mit eine HIV-Infektion assoziierte Erkrankungen oder Autoimmuner-krankungen in therapieren.

Kurze Beschreibung der Zeichnung:

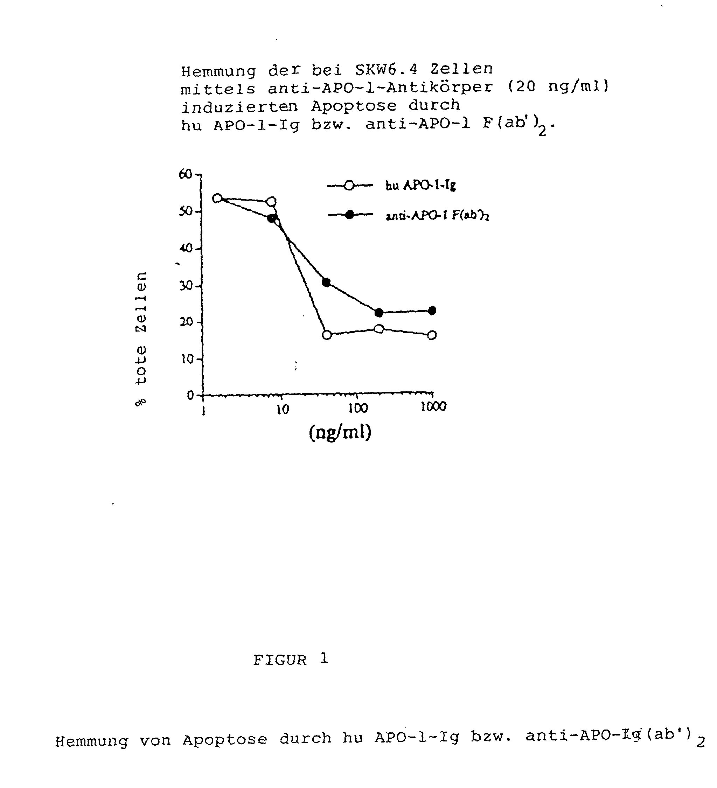

- Fig. 1: zeigt die Hemmung von Apoptose durch hu APO-1-Ig bzw. anti-APO-1F(ab')₂,

Die nachstehenden Beispiele erläutern die Erfindung.

### Beispiel 1: Herstellung eines Fusionsproteins, in dem eine extrazelluläre APO-1-Domäne über eine Antikörper-Hinge-Region an einen Fc-Teil eines humanen Antikörpers fusioniert ist.

Zur Herstellung vorstehenden Fusionsproteins wurden eine eine extrazelluläre APO-1-Domäne codierende cDNA (vgl. Oehm, A. et al., supra) und eine einen Fc-Teil eines humanen Antikörpers codierende cDNA (vgl. Dübel, S. et al., supra) einer üblichen PCR-Amplifikation unterzogen.

Als Primer für die cDNA der extrazellulären APO-1-Domäne wurden verwendet:
5' GCG AAG CTT GCC ACC ATG GTG GGC ATC TGG ACC CTC 3', wodurch eine Hind III-Stelle upstream einer vollständigen das Initiator-Methionin umgebenden Kozak-Consensus-Region eingeführt wird, und 5' GAC ACA ACA TTT GCG CTC GTT AGA TCT GGA TCC TTC 3', der für das 3'-Ende der extrazellulären APO-1-Domäne und die ersten 18 bp der Hinge-Region codiert.

Als Primer für die cDNA des Fc-Teils wurden verwendet:
5' GAG CGC AAA TGT TGT GTC GAG TGC 3', der für die ersten 18 bp der Hinge-Region und das 5'-Ende des Fc-Teils codiert, und 5' ATT AAG CAT TCT AGA TCA TTT ACC CGG AGA CAG GGA 3', der für das 3'-Ende des Fc-Teils codiert und eine Xbal-Stelle downstream des Stopcodons einführt.

Die erhaltenen PCR-Produkte wurden in einem "low melting point"-Agarosegel aufgetrennt und die Gelstückchen, die DNA-Moleküle richtiger Größe enthielten, wurden vereinigt. Mit einer Probe davon wurde eine weitere PCR-Amplifikation durchgeführt. Als Primer wurden nur jene vorstehenden verwendet, die dem 5'-Ende der extrazellulären APO-1-Domäne bzw. dem 3'-Ende des Fc-Teils entsprachen. Damit war es möglich, die extrazelluläre APO-1-Domäne über die gemeinsame Hinge-Region an den Fc-Teil zu fusieren. Es wurde ein "Fusions"-DNA-Fragment erhalten.

Dieses Fragment wurde mit HindIII und XbaI gespalten, über ein Agarosegel gereinigt und in dem Vektor pCDM8 kloniert. Die Sequenz des Inserts von pCDM8 wurde durch Dideoxynukleotid-Sequenzierung bestimmt.

### Beispiel 2: Hemmung von Apoptose durch hu APO-1-Ig bzw. anti-APO-1 F(ab')₂

SKW 6.4 Zellen (vgl. Oehm, A. et al., supra) sind Apoptose-positive Zellen, d.h. bei ihnen kann Apoptose induziert werden, z.B. durch Bindung eines anti-APO-1-Antikörpers.

SKW 6.4 Zellen wurden 24 h mit einem anti-APO-1-Antikörper in Gegenwart verschiedener Mengen des Fusionsproteins von Beispiel 1 (hu APO-1-Ig) bzw. von anti-APO-1-F(ab)₂ (vgl. vorstehend) inkubiert. Die Hemmung der Apoptose wurde bestimmt (vgl. Figur 1).

Es zeigte sich, daß das Fusionsprotein von Beispiel 1 und anti-APO-1-F(ab)₂ eine starke Apoptose-Hemmungs-Wirkung aufweisen. Diese ist bei dem Fusionsprotein besonders stark.

## Patentansprüche

1. Verwendung einer oder mehrerer den APO-1-Ligand hemmenden bzw. abfangenden Verbindungen, wobei die Verbindungen in einem Individuum nicht als fremd angesehen werden und ausgewählt sind aus:
- einem anti-APO-1-Ligand-Antikörper,
- einem APO-1,
- einer extrazellulären APO-1-Domäne,
- einer Verbindung mit mindestens einer extrazellulären APO-1-Domäne und einem Träger,
- einem eine APO-1-Ligand-Bindungsstelle aufweisenden Peptid, und
- einer Verbindung mit mindestens einem eine APO-1-Ligand-Bindungsstelle aufweisenden Peptid und einem Träger
zur Herstellung eines Arzneimittels zur Hemmung von Apoptose bei einer mit einer HIV-Infektion assoziierten Erkrankung oder Autoimmunerkrankung.

2. Verwendung nach Anspruch 1, wobei bei der Verbindung mit mindestens einer extrazellulären APO-1-Domäne und einem Träger die Domäne(n) und der Träger in einem Individuum nicht als fremd angesehen werden.

3. Verwendung nach Anspruch 2, wobei der Träger ein Protein ist.

4. Verwendung nach Anspruch 3, wobei das Protein ein Fc-Teil eines Antikörpers ist.

5. Verwendung nach einem der Ansprüche 3-4, wobei die Verbindung ein Fusionsprotein ist.

6. Fusionsprotein, bei dem eine extrazelluläre APO-1 Domäne über eine Antikörper-Hinge-Region an einen Fc-Teil eines humanen Antikörpers fusioniert ist, wobei die APO-1 Domäne aus den Aminosäuren 1 - 171 des humanen APO-1 Moleküls besteht.

## Claims

1. Use of one or more compounds inhibiting or catching the APO-1 ligand, wherein the compounds are not considered foreign in an individual and are selected from:
- an anti-APO-1 ligand antibody,
- an APO-1,
- an extracellular APO-1 domain,
- a compound having at least one extracellular APO-1 domain and a carrier,
- a peptide having an APO-1 ligand binding site, and
- a compound having at least one peptide including an APO-1 ligand binding site and a carrier,
for the production of a medicament for inhibiting apoptosis in the case of a disease associated with an HIV infection or an autoimmune disease.

2. Use according to claim 1, wherein with the compound having at least one extracellular APO-1 domain and a carrier the domain(s) and the carrier are not considered foreign in an individual.

3. Use according to claim 2, wherein the carrier is a protein.

4. Use according to claim 3, wherein the protein is an Fc portion of an antibody.

5. Use according to any of claims 3 to 4, wherein the compound is a fusion protein.

6. Fusion protein, in which an extracellular Apo-1 domain is fused to an Fc portion of a human antibody via an antibody hinge region, wherein the APO-1 domain consists of amino acids 1-171 of the human APO-1 molecule.

## Revendications

1. Utilisation d'un ou de plusieurs composés inhibiteurs ou capteurs du ligand de l'APO-1, composés qui ne sont pas considérés comme étrangers chez un individu et qui sont choisis entre :
- un anticorps anti-ligand APO-1,
- une APO-1,
- un domaine d'APO-1 extracellulaire,
- un composé ayant au moins un domaine d'APO-1 extracellulaire et un support,
- un peptide présentant un site de liaison du ligand APO-1, et
- un composé comprenant au moins un peptide présentant un site de liaison du ligand APO-1 et un support
pour la préparation d'un médicament destiné à inhiber l'apoptose dans une maladie associée à une infection par le virus VIH ou une maladie auto-immune.

2. Utilisation suivant la revendication 1, dans laquelle le ou les domaines et le support dont le composé comprenant au moins un domaine APO-1 extracellulaire et un support ne sont pas considérés comme étrangers chez un individu.

3. Utilisation suivant la revendication 2, dans laquelle le support est une protéine.

4. Utilisation suivant la revendication 3, dans laquelle la protéine est une partie Fc d'un anticorps.

5. Utilisation suivant l'une des revendications 3 et 4, dans laquelle le composé est une protéine de fusion.

6. Protéine de fusion, dans laquelle un domaine APO-1 extracellulaire est fusionné par l'intermédiaire d'une région charnière d'anticorps à une partie Fc d'un anticorps humain, le domaine APO-1 étant constitué des amino-acides 1-171 de la molécule d'APO-1 humaine.
